(19) Europäisches Patentamt / European Patent Office / Office européen des brevets

(11) **EP 4 390 831 B1**

(12) # EUROPÄISCHE PATENTSCHRIFT

(45) Veröffentlichungstag und Bekanntmachung des Hinweises auf die Patenterteilung:
**23.09.2026 Patentblatt 2026/39**

(21) Anmeldenummer: **22215016.1**

(22) Anmeldetag: **20.12.2022**

(51) Internationale Patentklassifikation (IPC):
***G06T 5/73*** *(2024.01)* ***G06T 5/10*** *(2006.01)*

(52) Gemeinsame Patentklassifikation (CPC):
**G06T 5/73; G06T 5/10;** G06T 2207/10116; G06T 2207/10121; G06T 2207/20016; G06T 2207/20192; G06T 2207/30101

(54) **VERFAHREN ZUR DIGITALEN BILDVERARBEITUNG VON MIT EINEM MEDIZINISCHEN BILDGEBUNGSGERÄT AUFGENOMMENEN MEDIZINISCHEN BILDERN UND MEDIZINISCHES GESAMTSYSTEM**

METHOD FOR DIGITAL IMAGE PROCESSING OF MEDICAL IMAGES RECORDED BY A MEDICAL IMAGING DEVICE AND A COMPLETE MEDICAL SYSTEM

PROCÉDÉ DE TRAITEMENT D’IMAGE NUMÉRIQUE D’IMAGES MÉDICALES PRISES PAR UN APPAREIL D’IMAGERIE MÉDICALE ET SYSTÈME MÉDICAL TOTAL

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(43) Veröffentlichungstag der Anmeldung:
**26.06.2024 Patentblatt 2024/26**

(73) Patentinhaber: **Siemens Healthineers AG**
**91301 Forchheim (DE)**

(72) Erfinder: **Fabricius, Uwe**
**91056 Erlangen (DE)**

(74) Vertreter: **Siemens Healthineers**
**Patent Attorneys**
**Postfach 22 16 34**
**80506 München (DE)**

(56) Entgegenhaltungen:
**US-A1- 2013 148 871**

- **STAHL M ET AL: "DIGITAL RADIOGRAPHY ENHANCEMENT BY NONLINEAR MULTISCALE PROCESSING", MEDICAL PHYSICS, AIP, MELVILLE, NY, US, vol. 27, no. 1, 1 January 2000 (2000-01-01), pages 56 - 65, XP000932832, ISSN: 0094-2405, DOI: 10.1118/1.598857**
- **GAMBARUTO A M: "Processing the image gradient field using a topographic primal sketch approach", INTERNATIONAL JOURNAL FOR NUMERICAL METHODS IN BIOMEDICAL ENGINEERING, JOHN WILEY & SONS, INC, HOBOKEN, USA, vol. 31, no. 3, 13 February 2015 (2015-02-13), pages n/a, XP072454835, ISSN: 2040-7939, DOI: 10.1002/CNM.2706**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die Erfindung betrifft ein Verfahren zur digitalen Bildverarbeitung von mit einem medizinischen Bildgebungsgerät aufgenommenen medizinischen Bildern eines Untersuchungsobjekts, wobei ein aufgenommenes medizinisches Bild mittels einer Frequenzzerlegung gemäß einer Gauß-Laplace-Zerlegung in eine Vielzahl von Ebenen zerlegt wird, gemäß dem Patentanspruch 1, sowie ein Computerprogrammprodukt gemäß dem Patentanspruch 12.

Im Rahmen der medizinischen Bildgebung und insbesondere Röntgenbildgebung, z.B. interventionellen Röntgenbildgebung, Fluoroskopie, Angiographie oder Radiographie, spielt die Bildqualität für eine Diagnosestellung eine große Rolle. Die (subjektive) Bildqualität von durch ein Bildgebungssystem aufgenommenen medizinischen Bildern eines Untersuchungsobjekts hängt maßgeblich von der Definition, insbesondere Kantendefinition, relevanter Strukturen und von der optischen Separierbarkeit der relevanten Strukturen vor einem Bildhintergrund ab. Sowohl die Kantendefinition als auch die Separierbarkeit der relevanten Strukturen werden durch Rauschen negativ beeinflusst, wobei eine niedrige Dosis (z.B. Röntgendosis) dies noch verstärkt. Eine klare, arktefaktfreie Abgrenzung relevanter Strukturen vom Bildhintergrund ist daher ein wichtiges Ziel.

Die Definition bzw. Hervorhebung von Strukturen bzw. Konturen im medizinischen Bild wird im einfachsten Fall durch ein Anheben höherer Bildfrequenzen (≙ Hochpassbild) bewerkstelligt, jedoch werden auf diese Weise alle entsprechenden Frequenzkomponenten, insbesondere auch das Rauschen, homogen im Bild verstärkt. Zudem ist dieser Ansatz begleitet von meist unerwünschten Effekten wie dem sogenannten Halo Effekt (hell überstrahlte Ränder) an den hervorgehobenen Kanten. Eine mögliche Maßnahme, den Rauschzuwachs zu reduzieren, ist eine Gewichtung bzw. Parametrierung durch ein Gradientenfeld, also z.B. eine Kantenverstärkung, die vom Gradientenfeld abhängig ist. Weitere Methoden zur Kantendefinition können kantenerhaltende Glättungen und bilaterale Filter sein (z.B. C. Cho und S. Lee, "Effective Five Directional Partial Derivatives-Based Image Smoothing and a Parallel Structure Design," in IEEE Transactions on Image Processing, vol. 25, no. 4, 2016, pp. 1617-1625, oder C. Tomasi und R. Manduchi, "Bilateral filtering for gray and color images", Sixth International Conference on Computer Vision, Bombay, 1998, pp. 839-846), welche jedoch hauptsächlich Artefakte erzeugende Wirkungsrichtungen minimieren, aber nicht die eigentliche Konturdefinition verbessern.

Eine andere Möglichkeit besteht darin, die visuelle Relevanz des Bildhintergrunds zu reduzieren. Hierfür können z.B. Frequenzfilter oder Grauwert-Mappings eingesetzt werden. Frequenzfilter wirken jedoch wiederum homogen auf den ganzen Frequenzbereich und generieren Halo-Effekte. Grauwert-Mappings sind nur bedingt geeignet, da hier Annahmen über die Grauwertbereiche von Bildinhalten gemacht werden. Fortgeschrittene Ansätze stützen sich auf eine Segmentierung, wobei hierbei Annahmen zu den zu separierenden Inhalten Voraussetzung sind, z.B. "röhrenförmige Struktur" (beispielsweise Anhebung gefäßähnlicher Strukturen mittels Frangi vesselness filter, siehe z.B. Frangi et al., "Multiscale vessel enhancement filtering" in: Medical Image Computing and Computer-Assisted Intervention - MICCAI'98, W.M. Wells, A. Colchester and S.L. Delp (Eds.), Lecture Notes in Computer Science, vol. 1496, Springer Verlag, Berlin, Germany, 1998, pp. 130-137).

Aus dem Artikel von M. Stahl et al.: "Digital Radiography Enhancement by nonlinear multiscale processing", MEDICAL PHYSICS, AIP, Melville, NY, US, Bd. 27, Nr. 1, 1. Januar 2000 (2000-01-01), Seiten 56-65, XP000932832, ISSN: 0094-2405, DOI: 10.1118/1.598857, ist ein Verfahren zur digitalen Bildverarbeitung von mit einem medizinischen Bildgebungsgerät aufgenommenen medizinischen Bildern eines Untersuchungsobjekts bekannt, wobei ein aufgenommenes medizinisches Bild mittels einer Frequenzzerlegung gemäß einer Gauß-Laplace-Zerlegung in eine Vielzahl von Ebenen zerlegt wird. Aus der Veröffentlichungsschrift US 2013/148871 A1 ist ein medizinisches Gesamtsystem zur digitalen Bildverarbeitung von durch ein Bildgebungsgerät aufgenommenen medizinischen Bildern eines Untersuchungsobjekts bekannt.

Es ist Aufgabe der vorliegenden Erfindung, ein Verfahren zur digitalen Bildverarbeitung für medizinische Bilder und insbesondere Röntgenbilder bereitzustellen, welches die Probleme des Standes der Technik überwindet und eine schärfere Kantendefinition ohne Rauschverstärkung und ohne Artefakte ermöglicht; des Weiteren ist es Aufgabe der Erfindung, ein für die Durchführung des Verfahrens geeignetes Computerprogrammprodukt bereitzustellen.

Die Aufgabe wird erfindungsgemäß gelöst durch ein Verfahren zur digitalen Bildverarbeitung von mit einem medizinischen Bildgebungsgerät aufgenommenen medizinischen Bildern eines Untersuchungsobjekts, wobei ein aufgenommenes medizinisches Bild mittels einer Frequenzzerlegung gemäß einer Gauß-Laplace-Zerlegung in eine Vielzahl von Ebenen zerlegt wird, gemäß dem Patentanspruch 1 und von einem Computerprogrammprodukt gemäß dem Patentanspruch 12. Vorteilhafte Ausgestaltungen der Erfindung sind jeweils Gegenstand der zugehörigen Unteransprüche.

Das erfindungsgemäße Verfahren zur digitalen Bildverarbeitung von mit einem medizinischen Bildgebungsgerät aufgenommenen medizinischen Bildern eines Untersuchungsobjekts, wobei ein aufgenommenes medizinisches Bild mittels einer Frequenzzerlegung gemäß einer Gauß-Laplace-Zerlegung in eine Vielzahl von Ebenen zerlegt wird, umfasst die folgenden Schritte: a) Bereitstellung je eines Bandpass- oder Tiefpassbildes zur Berechnung eines zugehöriger Gradientenfeldes für zumindest eine erste Ebene und für eine weitere Ebene der Frequenzzerlegung des

medizinischen Bildes, b) Bestimmung eines Skalarprodukts aus dem Gradientenfeld des Bandpass- oder Tiefpassbildes der ersten Ebene und dem, insbesondere hinsichtlich der Bildauflösung des Bandpass- oder Tiefpassbildes angepassten, Gradientenfeld des Bandpass- oder Tiefpassbildes der weiteren Ebene, c) Ermittlung eines Maskenbilds für die erste Ebene durch Normierung des Skalarproduktes, d) Integrierung des ermittelten Maskenbilds für die erste Ebene in eine Parametrierung zur Verarbeitung des Bandpass- oder Tiefpassbildes der ersten Ebene, und e) Durchführung einer Bildverarbeitungsoperation mit dem in die Parametrierung integrierten Maskenbild.

Die Idee des Verfahrens basiert auf der Erkenntnis, dass relevante Bildinhalte eines medizinischen Bildes auf mehrere, insbesondere benachbarte, Ebenen einer Frequenzzerlegung verteilt sind, wohingegen Rauschen sich nicht konsistent über Ebenen hinweg manifestiert. Mit dieser Erkenntnis wird im Rahmen des Verfahrens ein Maskenbild für zumindest eine Ebene der Frequenzzerlegung bestimmt, welches eine Kombination aus zwei Gradientenfeldern von Bandpässen oder Tiefpässen unterschiedlicher Ebenen verwendet. Grundsätzlich ist ein Gradientenfeld ein Feld aus Vektoren, z.B. im Falle des medizinischen Bildes zweidimensionalen Vektoren. Bei der Frequenzzerlegung eines Bildes sind diese Vektoren bei relevanten Strukturen über benachbarte Ebenen hinweg ausgerichtet und im Gegensatz dazu bei Rauschen zufällig verteilt.

Wird das derart bestimmte Maskenbild bei der Verarbeitung der zugehörigen Ebene in die Parametrierung eingebaut, so kann damit eine gleichzeitig geschärfte als auch artefaktreduzierte Kantendefinition erzielt werden. Dies ist unter anderem deshalb der Fall, weil durch das Maskenbild genau die Kantenbereiche identifiziert und ausgewählt werden, für die eine solche Kantendefinition möglich ist. Durch das Verfahren werden relevante Strukturen gegenüber dem Bildhintergrund besser erkennbar, Artefakte wie Halo Effekte werden vermieden. Es muss keine vorherige Annahme von zu erwartenden Strukturen in die Bildverarbeitung eingespeist werden, um gute Ergebnisse zu erzielen. Bereiche des medizinischen Bildes ohne Konturen können außerdem geglättet werden. Insgesamt wird die Bildqualität medizinischer Bilder signifikant verbessert, was wiederum zu verbesserten Diagnosestellungen und besseren Therapieerfolgen und damit einer besseren Patientenversorgung führt.

Das Prinzip einer Frequenzzerlegung eines Bildes nach Gauß-Laplace ist bekannt, siehe z.B. P.J. Burt, E.H. Adelson, "The Laplacian Pyramid as a Compact Image Code", 1983, IEEE Transactions on Communications, vol. com-31, no. 4. Um eine Gauß-Laplace-Pyramide zu erhalten kann zunächst eine Gauß-Pyramide ermittelt werden. Dies kann folgendermaßen beschrieben werden: Das originale medizinische Bild ist die unterste Pyramidenstufe und die nächste Stufe wird über eine Tiefpassfaltung (Faltung mit Gauß-Glocke) und Halbierung (nicht zwingend, kann auch weggelassen werden) der Stützstellen errechnet. Dies wird so lange wiederholt bis das oberste Bild eine Größe von $1 \times 1$ Pixel erreicht. Aus der Gauß-Pyramide kann dann eine Laplace-Pyramide ermittelt werden indem aus der Differenz zweier benachbarter Gaußebenen eine Laplace-Ebene bestimmt wird. Häufig können die Gaußebenen und Laplace-Ebenen auch parallel ermittelt werden. Damit die Frequenzbilder benachbarter Gaußebenen die gleiche Größe aufweisen, wird eine Interpolation auf die höhere Ebene (mit geringerer Auflösung) angewandt. Die einzelnen Laplace-Ebenen der Laplace-Pyramide repräsentieren die Bildinhalte in dem der jeweilen Ebene zugeordneten räumlichen Frequenzbereich (Bandpassbilder).

Das Maskenbild wird durch eine geeigneten Normierung der Pixelwerte des jeweiligen Skalarprodukts auf das Intervall [0,1] bestimmt.

Unter der ersten Ebene soll im Zusammenhang mit dem Verfahren i.A. nicht die gemäß Reihenfolge der Frequenzzerlegung erste Ebene verstanden werden, sondern lediglich eine beliebige ausgewählte Ebene, für die das Verfahren durchgeführt wird. Es kann jedoch sinnvoll sein, z.B. für den Fall, dass lediglich ein Maskenbild für das ganze medizinische Bild ermittelt und eingesetzt wird, als erste Ebene die gemäß Reihenfolge der Frequenzzerlegung unterste ("erste") Ebene zu verwenden.

Im Anschluss an die im Verfahren beschriebene Bildverarbeitung erfolgt üblicherweise eine Rekombination der prozessierten Ebenen entsprechend ihrer Zerlegung, um ein Ergebnisbild zu erhalten.

Nach einer Ausgestaltung der Erfindung wird die weitere Ebene von einer zu der ersten Ebene benachbarten Ebene gebildet, insbesondere einer benachbarten Ebene mit geringerer Bildauflösung (i.A. gemäß Pyramide darüber liegende Ebene). Handelt es sich bei der ersten Ebene also z.B. um die unterste Ebene $E_1$, wird als weitere Ebene z.B. die darüberliegende zweitunterste Ebene $E_2$ verwendet. Werden hierfür im Rahmen des Verfahrens benachbarte Ebenen verwendet, so ist sichergestellt, dass relevante Strukturen geschärft werden, da diese insbesondere bei nebeneinander liegenden Ebenen konsistent erkennbar sind, und dass Rauschen nicht verstärkt wird, da für Rauschen keine solche Konsistenz vorliegt.

Nach einer weiteren Ausgestaltung der Erfindung werden die Schritte a bis d für mindestens zwei verschiedene Ebenen durchgeführt und wird der Schritt e mit mindestens zwei in die jeweilige Parametrierung integrierten Maskenbildern durchgeführt. Je mehr Maskenbilder desto schärfer kann die Kantendefinition durchgeführt werden und desto besser wird voraussichtlich die Bildqualität ausfallen. Insbesondere werden die Schritte a bis d für eine Vielzahl von Ebenen oder sogar allen bis auf die oberste Ebene der Frequenzzerlegung durchgeführt und wird der Schritt e mit der Vielzahl von integrierten Maskenbildern durchgeführt. Auf diese Weise ergibt sich eine optimal geschärfte Struktur und damit optimale Bildqualität des medizinischen Bildes.

Nach einer weiteren Ausgestaltung der Erfindung wird ein Bandpassbild $v_L$ für die erste Ebene (als erste ausgewählte Ebene) der Frequenzzerlegung mittels der folgenden Formel bestimmt: $v_L := (\mathbb{1}_L - I_L T_{L+1}^L R_L^{L+1} S_L) \cdot u_L$ , wobei L die Nummerierung der ersten Ebene bezüglich der Reihenfolge der Frequenzzerlegung, $R_L^{L+1}$ ein auflösungsreduzierender Operator, $T_{L+1}^L$ ein auflösungsvergrößernder Operator, S ein Glättungsoperator, I ein Interpolationsoperator, $\mathbb{1}_L$ ein Identitätsoperator und $u_L$ das jeweilige Eingangsbild der ersten Ebene sind. Bei der Formel handelt es sich um eine gängige Darstellung zur Bestimmung eines Bandpassbildes einer Ebene der Laplace-Pyramide.

Nach einer weiteren Ausgestaltung der Erfindung wird das Gradientenfeld des Bandpass- oder Tiefpassbildes der weiteren Ebene, insbesondere der zu der ersten Ebene benachbarten Ebene, hinsichtlich der Bildauflösung des Bandpass- oder Tiefpassbildes angepasst, insbesondere unter Verwendung eines auflösungsvergrößernden Operators ( $T_{L+1}^L$ ). Dies ist sinnvoll, um das Skalarprodukt auf einfache Weise bestimmen zu können.

Nach einer weiteren Ausgestaltung der Erfindung wird das Skalarprodukt mittels der Formel $\langle \nabla v_L, I_L T_{L+1}^L \nabla v_{L+1} \rangle$ oder mittels der Formel $\langle \nabla v_L, I_L T_{L+1}^L \nabla u_{L+1} \rangle$ gebildet, wobei $\nabla v_L$ das Gradientenfeld des Bandpassbildes der ersten Ebene, $\nabla v_{L+1}$ das Gradientenfeld des Bandpassbildes der weiteren Ebene, $T_{L+1}^L$ ein auflösungsvergrößernder Operator, $I_L$ ein Interpolationsoperator und $\nabla u_{L+1}$ das Gradientenfeld des jeweiligen Eingangsbilds der weiteren Ebene sind. Hierbei handelt es sich um eine übliche Darstellungen für ein Skalarprodukt, wobei die Bildauflösung der benachbarten Bandpassbildes (mit der niedrigeren Auflösung) an die höhere Auflösung angepasst wird, um eine schnelle und einfache Bestimmung möglich zu machen.

Nach einer weiteren Ausgestaltung der Erfindung wird das Maskenbild $\Theta_L$ aus dem Skalarprodukt anhand der folgenden Formel bestimmt: $\Theta_L := \overline{\langle \nabla v_L, I_L T_{L+1}^L \nabla v_{L+1} \rangle}$ , wobei $\nabla v_L$ das Gradientenfeld des Bandpassbildes der ersten Ebene, $\nabla v_{L+1}$ das Gradientenfeld des Bandpassbildes der weiteren Ebene, $T_{L+1}^L$ ein auflösungsvergrößernder Operator und $I_L$ ein Interpolationsoperator sind. Das Maskenbild entsteht damit aus einer Normierung des Skalarprodukts, so dass dessen Wertebereich [0,1] ist. Hohe Werte nahe 1 markieren die Kanten relevanter Strukturen bezogen auf das Bandpassbild $v_L$, bei niedrigen Werten sind keine relevanten Strukturen zu finden.

In vorteilhafter Weise ist das entsprechende Maskenbild ausgebildet, eine schärfere Definition von Konturen von in dem medizinischen Bild vorhandenen Kanten bzw. relevanten Strukturen (z.B. Objekt wie Organ, Knochen oder Blutgefäß) zu bewirken. Relevante Strukturen sind auf mehreren Ebenen der Frequenzzerlegung vorhanden und somit sind an den Positionen der relevanten Strukturen hohe Werte nahe 1 für das Maskenbild zu finden. Eine entsprechende Bildverarbeitung in dem Bereich der Strukturen bewirkt dort eine schärfere Definition der Kanten. In vorteilhafter Weise ist das Maskenbild ausgebildet, eine Glättung von Bereichen des medizinischen Bildes ohne Konturen oder mit wenig ausgeprägten Konturen zu bewirken, was sich an diesen Positionen durch die niedrigen Werte des Maskenbildes nahe 0 ergibt.

Nach einer weiteren Ausgestaltung der Erfindung wird zur Durchführung des Verfahrens zumindest ein vortrainierter maschinenlernender Algorithmus eingesetzt. Dieser kann besonders geeignet sein, um eine schnelle und einfache Durchführung des Verfahrens zu erreichen.

In einem Beispiel, welches das Verständnis der Erfindung erleichtert, ist auch ein medizinisches Gesamtsystem zur digitalen Bildverarbeitung von durch ein medizinisches Bildgebungsgerät aufgenommenen medizinischen Bildern eines Untersuchungsobjekts gemäß dem Verfahren umfasst, welches eine Bereitstellungseinheit zur Bereitstellung zumindest eines medizinischen Bildes, eine Bildverarbeitungseinheit zur Bearbeitung und zur Frequenzzerlegung von medizinischen Bildern, insbesondere mittels zumindest eines Bildverarbeitungsalgorithmus, eine Steuerungseinheit zur Ansteuerung des medizinischen Gesamtsystems, und eine Ausgabeeinheit zur Ausgabe des verarbeiteten medizinischen Bildes aufweist. Die Bildverarbeitungseinheit ist insbesondere dazu ausgebildet, ein Bandpass- oder Tiefpassbild und ein zugehöriges Gradientenfeld für zumindest eine erste Ebene und für eine weitere Ebene der Frequenzzerlegung des medizinischen Bildes zu bestimmen, ein Skalarprodukts aus dem Gradientenfeld des Bandpass- oder Tiefpassbildes der ersten Ebene und dem Gradientenfeld des Bandpass- oder Tiefpassbildes der weiteren Ebene zu bestimmen, ein Maskenbild für die erste Ebene durch Normierung des Skalarproduktes zu ermitteln, dieses in eine Parametrierung des Bandpass- oder Hoch-passbildes der ersten Ebene zu integrieren und derart eine Bildverarbeitungsoperation durchzuführen. Insbesondere sind ein oder mehrere Algorithmen dazu ausgebildet, die Schritte des Verfahrens auszuführen.

Außerdem ist ein Computerprogrammprodukt mit zumindest einem Algorithmus zur digitalen Bildverarbeitung zur Durchführung des Verfahrens umfasst.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:

FIG 1 eine Abfolge der Schritte des erfindungsgemäßen Verfahrens;

FIG 2 eine Ansicht eines beispielhaften medizinischen Bildes;

FIG 3 eine Ansicht eines aus einer Frequenzzerlegung des medizinischen Bildes in FIG 2 bestimmten Maskenbildes;

FIG 4 eine Ansicht eines ursprünglich unverarbeiteten medizinischen Bildes und zweier nach Verwendung des Verfahrens resultierender Bilder;

FIG 5 eine Ansicht einer typischen Kante in einem medizinischen Bild und einer dazu gehörigen auf Hochpass und Tiefpass reduzierten Frequenzzerlegung;

FIG 6 eine Ansicht der Kante von FIG 5, welche durch eine Kantenanhebung gemäß dem Stand der Technik verstärkt wurde;

FIG 7 eine Ansicht der gemäß dem Verfahren geschärften Kante von FIG 5 sowie eines zugehörigen Maskenbildes; und

FIG 8 eine Ansicht eines medizinischen Gesamtsystems.

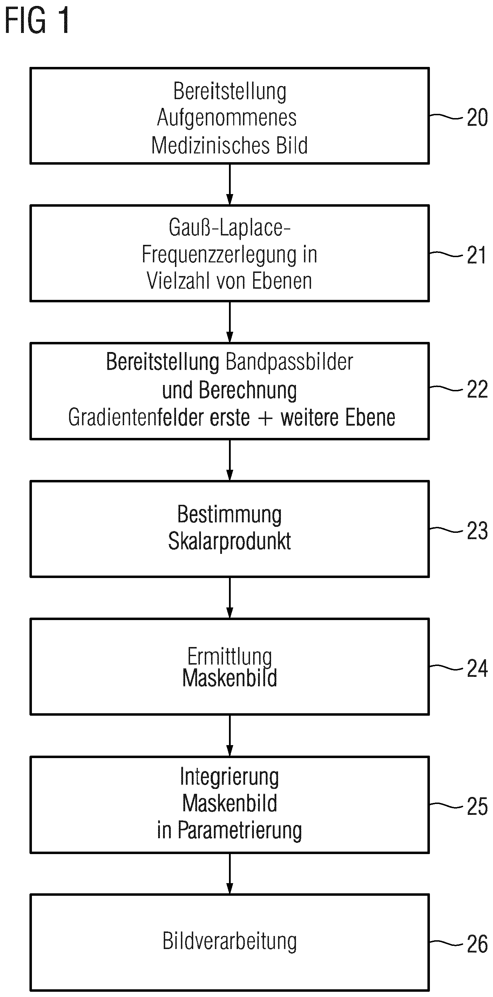

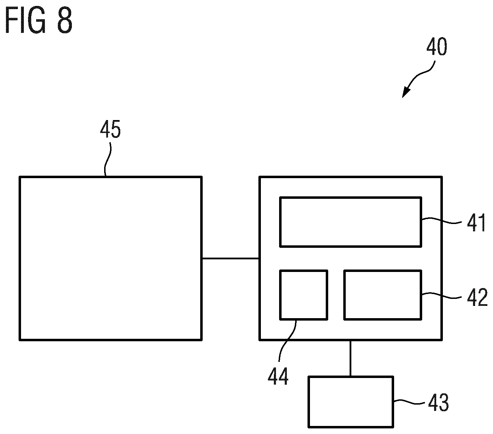

In der FIG 1 sind Schritte des Verfahrens zur digitalen Bildverarbeitung von mit einem medizinischen Bildgebungsgerät aufgenommenen medizinischen Bildern eines Untersuchungsobjekts gezeigt. Die medizinischen Bilder können z.B. von einem Röntgengerät aufgenommen werden (z.B. 2D-Projektionsbilder). In der FIG 8 ist ein Gesamtsystem 40 zur Durchführung des Verfahrens gezeigt. Durch das Verfahren ist es möglich, artefaktfrei relevante Strukturen wie Organe oder andere Objekte schärfer zu definieren und Rauschen zu reduzieren oder zu glätten und auf diese Weise eine hohe Bildqualität zu erzielen. Besonders bei medizinischen Bildern bzw. Röntgenbildern ist eine hohe Bildqualität notwendig, um eine gute Diagnosestellung gewährleisten zu können. Bei einer Fluoroskopieüberwachung während eines interventionellen Eingriffes ist es wichtig, durch die permanente Röntgenstrahlung eine möglichst geringe Dosis zu applizieren, was auch zu hohem Rauschen führen kann.

Der hier beschriebene Ansatz basiert auf einer Frequenzzerlegung des medizinischen Bildes in einer Laplace-Pyramide und der vergleichsweisen offenen Annahme, dass relevante Bildinhalte auf mehrere, insbesondere benachbarte, Ebenen (Band- oder Tiefpassbilder) dieser Zerlegung verteilt sind, wohingegen Rauschen sich nicht konsistent über Ebenen hinweg manifestiert.

In einem ersten Schritt 20 wird ein medizinisches Bild, z.B. ein (2D-)Röntgenbild, bereitgestellt. Das medizinische Bild kann dabei entweder aus einem Speicher oder einer Datenbank entnommen oder auch direkt unter Verwendung eines dem Gesamtsystem 40 zugeordneten Bildgebungsgeräts (z.B. Angiographieröntgengerät, festinstalliertes oder mobiles C-Bogenröntgengerät, CToder ähnliches) aufgenommen und zum Beispiel von einer Bereitstellungseinheit 41 zur Verfügung gestellt werden. Das medizinische Bild wurde durch Röntgendurchleuchtung eines Untersuchungsobjekts erstellt, z.B. eines Organs oder Körperteils eines Patienten. Es kann sich hier z.B. um ein Herz, ein Blutgefäß, eine Leber, eine Lunge, eine Wirbelsäule oder einen Knochen eines Patienten handeln. Das medizinische Bild kann noch unverarbeitet sein oder bereits teilweise verarbeitet sein. Medizinische Bilder werden im Allgemeinen nach der Aufnahme durch eine Reihe von Bildverarbeitungsmethoden nachbearbeitet, z.B. um die Bildqualität zu steigern und Bewegungen, Artefakte oder Rauscheffekte zu korrigieren.

In einem zweiten Schritt 21 wird das medizinische Bild mittels einer Gauß-Laplace-Frequenzzerlegung in eine Vielzahl von Ebenen zerlegt. Eine derartige Frequenzzerlegung ist aus dem Stand der Technik bekannt und kann standardmäßig durchgeführt werden. Die Reihenfolge kann zum Beispiel derart sein, dass zunächst eine Gauß-Zerlegung und dann daraus eine Laplace-Zerlegung durchgeführt werden. Dies kann sowohl grundsätzlich (also zunächst die gesamte Gauß-Zerlegung und anschließend die Laplace-Zerlegung) als auch für einzelne Ebenen (zuerst die Gaußebene dann die entsprechende Laplace-Ebene) durchgeführt werden. Häufig werden beide Zerlegungen parallel durchgeführt, z.B. Ebene für Ebene. Die Ergebnisse der Gauß-Zerlegung (Tiefpassbilder) können bei Bedarf beibehalten oder auch verworfen werden, wenn sie nicht benötigt werden. Zur Durchführung der Gauß-Laplace-Frequenzzerlegung kann eine Bildverarbeitungseinheit 42 verwendet werden.

In einem dritten Schritt 22 wird für eine erste Ebene und für eine weitere Ebene der Frequenzzerlegung je ein gemäß der zuvor durchgeführten Zerlegung berechnetes Bandpassbild bereitgestellt und daraus ein zugehöriges Gradientenfeld berechnet. Hierbei soll die Bezeichnung als erste Ebene nicht auf die Reihenfolge der Frequenzzerlegung (also nicht die "unterste" Ebene der Frequenzzerlegung) bezogen sein, sondern allgemein für die jeweilige für das Verfahren ausgewählte Ebene stehen. Es wird deshalb im Folgenden der Index L benutzt, die benachbarte weitere Ebene

wird mit L+1 nummeriert. Bei einer Frequenzzerlegung nach Laplace in N Ebenen $E_i$ ($i \in [1...N]$) kann der Index L also für eine Ebene $E_L$ der Ebenen $E_1$ bis $E_{N-1}$ stehen.

In der Laplace-Frequenzzerlegung soll $v_L$ das Bandpassbild auf der ersten Ebene und $u_L$ das zugehörige Eingangsbild (der Gauß-Pyramide) sein, so dass

$$v_L := (\mathbb{1}_L - I_L T_{L+1}^L R_L^{L+1} S_L) \cdot u_L$$

gilt. T und R sind Operatoren für eine Auflösungsvergrößerung (T, UpSampling) bzw. Auflösungsreduzierung (R, DownSampling) zwischen den Ebenen L und L+1, und I und S geeignete Faltungsoperatoren für Interpolation (I) und Glättung (S). Insbesondere ist $u_1$ das unverarbeitete Eingangsbild, also das ursprünglich aufgenommene medizinische Bild, vor der Zerlegung. $\mathbb{1}$ ist ein Identitätsoperator. Die entsprechenden Gradientenfelder sind $\nabla v_L$ und $\nabla V_{L+1}$. Der dritte Schritt 22 kann ebenfalls von der Bildverarbeitungseinheit 42 oder einer weiteren Recheneinheit durchgeführt werden. Es können ein oder mehrere Algorithmen hierfür verwendet werden. Der zweite Schritt 21 und der dritte Schritt 22 können auch zusammen durchgeführt werden.

In einem vierten Schritt 23 wird ausgehend von den Gradientenfeldern $\nabla v_L$ und $\nabla V_{L+1}$ das Skalarprodukt $\langle \nabla v_L, I_L T_{L+1}^L \nabla v_{L+1} \rangle$ aus den Gradientenfeldern der ersten Ebene und ihrer benachbarten Ebene berechnet. Die Operatoren I (Interpolation) und T (Auflösungsvergrößerung) wirken hierbei komponentenweise auf das Gradientenfeld der weiteren Ebene. Durch die Auflösungsvergrößerung wird die Auflösung des Gradientenfelds der L+1-ten Ebene auf die der L-ten Ebene angehoben. Abhängig vom Design der Pyramide und dem Ebenenindex L kann auch die Verwendung von $\langle \nabla v_L, I_L T_{L+1}^L \nabla u_{L+1} \rangle$ vorteilhaft sein. Zur weiteren Beschreibung beschränken wir uns jedoch auf erstere Variante. Die Berechnung des Skalarprodukts kann z.B. wiederum mittels der Bildverarbeitungseinheit 42 oder einer anderen Recheneinheit durchgeführt werden.

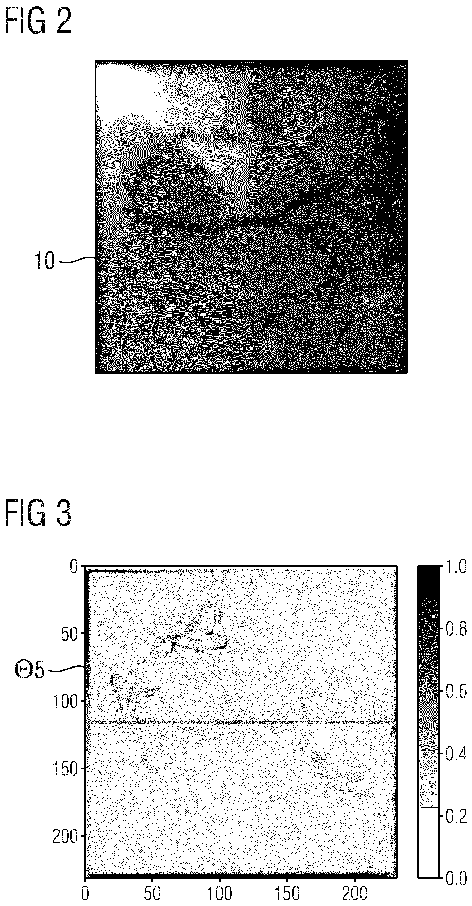

In einem fünften Schritt 24 wird anschließend aus dem im vierten Schritt 23 berechneten Skalarprodukt durch eine geeignete Normierung ein Maskenbild $\Theta_L := \overline{\langle \nabla v_L,\ I_L\ T_{L+1}^L\ \nabla v_{L+1} \rangle}$ für die erste Ebene (Index L) berechnet. Das Maskenbild besitzt einen Wertebereich [0,1]. Höhere Werte über 0,5 markieren insbesondere relevante Strukturen bzw. Objekte und deren Kanten bezogen auf das Bandpassbild $v_L$. Niedrige Werte im Bereich von 0 enthalten keine relevanten Strukturen, können aber insbesondere Rauschen enthalten. In den Figuren 2 und 3 ist ein Beispiel für ein medizinisches Bild 10 eines Gefäßbaumes (FIG 2) und eines hierzu ermittelten Maskenbildes $\Theta_5$ (FIG 3) der fünftuntersten Laplace-Ebene (nur beispielhaft) gezeigt. Die Strukturen der Gefäßränder sind deutlich erkennbar, der Hintergrund weist einen Wertebereich nahe 0 auf.

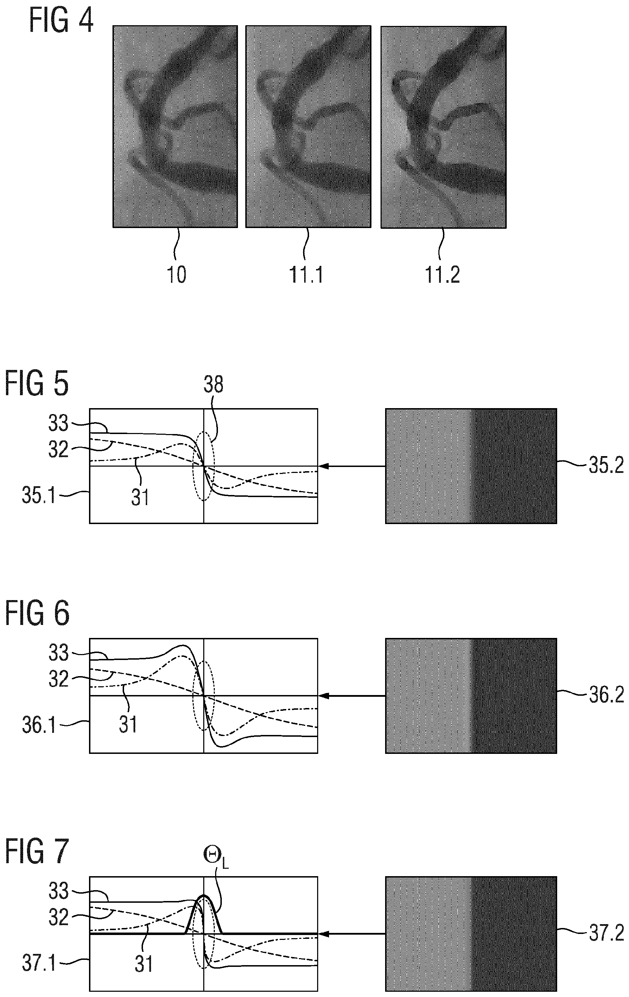

Das Maskenbild $\Theta_L$ wird anschließend in einem sechsten Schritt 25 in die Parametrierung der Verarbeitung des ersten Bandpassbildes $v_L$ integriert. Das bedeutet, dass das Maskenbild als Parameter bzw. Verstärkungsfaktor in die Verarbeitung des Bandpassbildes $v_L$ einfließt. Im vorliegenden Fall besteht die Wirkung des Maskenbilds darin, dass die Pixel des Bandpassbildes $v_L$ in den Bereichen mit hohen Werten des Maskenbildes verstärkt werden im Vergleich zu denen mit niedrigen Werten (siehe auch FIG 7, Erläuterung weiter unten).

Mit dem so eingebauten Maskenbild findet dann in einem siebten Schritt 26 eine Bildverarbeitungsoperation statt. Diese kann auch weitere Parameter enthalten (z.B. Rauschreduzierung, Glättung, Artefaktreduzierung, Korrektur usw.). Durch das Maskenbild werden im Rahmen der Bildverarbeitungsoperation z.B. die Konturen schärfer definiert, ohne Artefakte wie Halos zu erzeugen oder die Kanten zu glätten. Es können so auch konturenfreie Bereiche geglättet werden, wodurch das Rauschen deutlich reduziert wird ohne Kanten zu verwischen.

Im Anschluss an die im Verfahren beschriebene Bildverarbeitung erfolgt üblicherweise eine Rekombination der prozessierten Ebenen entsprechend ihrer Zerlegung, um ein Ergebnisbild zu erhalten.

In der FIG 4 ist ein medizinisches Bild 10 eines weiteren Gefäßbaums gezeigt sowie zwei mittels des Verfahrens bearbeitete medizinische Bilder 11.1 und 11.2. Die gemäß Verfahren bearbeiteten Bilder 11.1 und 11.2 zeigen die Bildqualitätsverbesserung durch die verstärkte Kantendefinierung bei zwei beispielhaften Parametrierungen. Bei dem mittleren Bild 11.1 ist dabei eine leicht verstärkte Kantendefinierung und bei dem rechten Bild 11.2 eine sehr starke Kantendefinierung zu erkennen.

Der dritte Schritt 22, der vierte Schritt 23, der fünfte Schritt 24 und der sechste Schritt 25 können bei Bedarf auch für eine zweite Ebene (z.B. Index M, ebenfalls von 1 bis N-1 möglich, wobei $L \neq M$; benachbarte Ebene M+1) wiederholt werden, so dass dann ein weiteres Maskenbild $\Theta_M$ für die zweite Ebene ermittelt wird. Der siebte Schritt 26, also die Bildverarbeitung wird dann durchgeführt mit in die Parametrierung der Bandpassbilder $v_L$ und $v_M$ ihrer jeweiligen Ebenen (Indices L und M) integrierten Maskenbildern $\Theta_L$ und $\Theta_M$.

Wahlweise können der dritte Schritt 22, der vierte Schritt 23, der fünfte Schritt 24 und der sechste Schritt 25 auch für eine Vielzahl von Ebenen oder alle Ebenen (abgesehen von der obersten, da diese keinen weiteren Nachbarn

aufweist) durchgeführt werden. Dann können die so erhaltenen Maskenbilder in die Parametrierung der Bandpassbilder ihrer jeweiligen Ebenen integriert werden und eine Bildverarbeitung mit den integrierten Maskenbildern durchgeführt werden.

In einigen Fällen kann anstelle der zu der ersten Ebene benachbarten weiteren Ebene auch eine nicht-benachbarte weitere Ebene verwendet werden, zum Beispiel der übernächste Nachbar (L+2). Es sollte jedoch immer eine maximal die gleiche oder eine geringere Auflösung aufweisende Ebene verwendet werden, nicht eine Ebene mit einer größeren Auflösung. Grundsätzlich können Ebenenpaare $E_i$ und $E_{i+d}$ mit $d > 0$ verwendet werden.

Um die Wirkungsweise des Verfahrens hinsichtlich der verbesserten Kantendefinierung zu illustrieren, ist in der FIG 5 eine übliche verschwommene Strukturkante in einer auf das Minimum eines Hoch- und eines Tiefpasses reduzierten erste Frequenzzerlegung 35.1 und deren ungefähr entsprechende erste Grauwertdarstellung 35.2 gezeigt. Die erste Frequenzzerlegung 35.1 zeigt die Hochpasskurve 31 und die Tiefpasskurve 32 entlang der horizontalen Schnittlinie 33 durch die Strukturkante. Eine visuell scharfe Definition der Kante hängt von der Steilheit der Schnittlinie 33 im gestrichelt markierten Kantenbereich 38 ab. Eine kantenerhaltende Glättung hat an diesem Bild keinen Effekt, da die Strukturkante bereits perfekt glatt entlang ihrer senkrechten Ausrichtung ist.

In der FIG 6 zeigt die zweite Frequenzzerlegung 36.1 das Resultat eines bekannten Kantenanhebungsverfahrens, welches die Hochpasskurve 31 deutlich anhebt und die Steilheit der Schnittlinie 33 nahe des Kantenmittelpunkts verstärkt. Dadurch kommt es jedoch zu einem unerwünschten Halo Effekt, der in der zweiten Grauwertdarstellung 36.2 erkennbar ist.

In der FIG 7 ist in einer dritten Frequenzzerlegung 37.1 das Ergebnis des beschriebenen Verfahrens zu sehen, wobei gleichzeitig der Wertebereich des Maskenbildes $\Theta$ entlang der Kante sichtbar ist. Insgesamt wurde eine Verstärkung der Hochpasskurve 31 auf den für die scharfe Definition der Kante relevanten Bereich eingeschränkt. Dadurch konnte die Breite des Übergangs zwischen den beiden Grauwerten der dritten Grauwertdarstellung 37.2 reduziert und die üblichen Artefakte, wie Halos, geringgehalten werden.

Das beschriebene Verfahren kann je nach Interpretation von $\Theta$ variabel eingesetzt werden zur Glättung und/oder Kantendefinition des medizinischen Bildes. Kanten werden dabei nicht wie bei üblichen Verfahren verstärkt oder kantenerhaltend geglättet, sondern die Konturen werden schärfer definiert. Durch die Verwendung einer Kombination zweier Gradientenfelder von Band- und/oder Tiefpässen unterschiedlicher Frequenzbereiche werden nicht nur Konturen relevanter Objekte detektiert, sondern insbesondere genau die Kantenbereiche innerhalb des höherfrequenten Bandpassbildes identifiziert, die zur schärferen, aber artefaktreduzierten, Kantendefinition modifiziert werden können.

In der FIG 8 ist das medizinische Gesamtsystem 40 zur Durchführung des Verfahrens gezeigt. Dieses umfasst eine Bereitstellungseinheit 44 zur Bereitstellung zumindest eines medizinischen Bildes, eine Bildverarbeitungseinheit 42 zur Bearbeitung und zur Frequenzzerlegung von medizinischen Bildern, insbesondere mittels zumindest eines Bildverarbeitungsalgorithmus, eine Steuerungseinheit 41 zur Ansteuerung des medizinischen Gesamtsystems und eine Anzeigeeinheit 43 zur Ausgabe des verarbeiteten medizinischen Bildes. Dem Gesamtsystem 40 ist ein Röntgengerät 45 zugeordnet.

Unabhängig vom grammatikalischen Geschlecht eines bestimmten Begriffes sind Personen mit männlicher, weiblicher oder anderer Geschlechteridentität mit umfasst.

Die Erfindung lässt sich in folgender Weise kurz zusammenfassen: Das erfindungsgemäße Verfahren zur digitalen Bildverarbeitung von mit einem medizinischen Bildgebungsgerät aufgenommenen medizinischen Bildern eines Untersuchungsobjekts, wobei ein aufgenommenes medizinisches Bild mittels einer Frequenzzerlegung gemäß einer Gauß-Laplace-Zerlegung in eine Vielzahl von Ebenen zerlegt wird, umfasst die folgenden Schritte: a) Bereitstellung je eines Bandpass- oder Tiefpassbildes zur Berechnung eines zugehörigen Gradientenfeldes für zumindest eine erste Ebene und für eine weitere Ebene der Frequenzzerlegung des medizinischen Bildes, b) Bestimmung eines Skalarprodukts aus dem Gradientenfeld des Bandpass- oder Tiefpassbildes der ersten Ebene und dem, insbesondere hinsichtlich der Bildauflösung des Bandpass- oder Tiefpassbildes angepassten, Gradientenfeld des Bandpass- oder Tiefpassbildes der weiteren Ebene, c) Ermittlung eines Maskenbilds für die erste Ebene durch Normierung des Skalarproduktes, d) Integrierung des ermittelten Maskenbilds für die erste Ebene in eine Parametrierung zur Verarbeitung des Bandpass- oder Hoch-passbildes der ersten Ebene , und e) Durchführung einer Bildverarbeitungsoperation mit dem in die Parametrierung integrierten Maskenbild.

## Patentansprüche

1. Computerimplementiertes Verfahren zur digitalen Bildverarbeitung von mit einem medizinischen Bildgebungsgerät aufgenommenen medizinischen Bildern eines Untersuchungsobjekts, wobei ein aufgenommenes medizinisches Bild (10) mittels einer Frequenzzerlegung gemäß einer Gauß-Laplace-Zerlegung in eine Vielzahl von Ebenen ($E_i$) zerlegt wird, mit den folgenden Schritten:

a. Bereitstellung je eines Bandpass- oder Tiefpassbildes zur Berechnung eines zugehörigen Gradientenfeldes für zumindest eine erste Ebene ($E_L$) und für eine weitere Ebene ($E_{L+1}$) der Frequenzzerlegung des medizinischen Bildes (10),
b. Bestimmung eines Skalarprodukts aus dem Gradientenfeld ($\nabla v_L$) des Bandpass- oder Tiefpassbildes der ersten Ebene ($E_L$) und dem, insbesondere hinsichtlich der Bildauflösung des Bandpass- oder Tiefpassbildes angepassten, Gradientenfeld ($\nabla v_{L+}1$) des Bandpass- oder Tiefpassbildes der weiteren Ebene ($E_{L+1}$),
c. Ermittlung eines Maskenbilds ($\Theta_L$) für die erste Ebene ($E_L$) durch Normierung des Skalarproduktes,
d. Integrierung des ermittelten Maskenbilds ($\Theta_L$) für die erste Ebene ($E_L$) in eine Parametrierung zur Verarbeitung des Bandpass- oder Tiefpassbildes der ersten Ebene ($E_L$), und
e. Durchführung einer Bildverarbeitungsoperation mit dem in die Parametrierung integrierten Maskenbild ($\Theta_L$).

**2.** Verfahren nach Anspruch 1, wobei die weitere Ebene ($E_{L+1}$) von einer zu der ersten Ebene benachbarten Ebene ($E_{L+1}$) gebildet wird, insbesondere einer benachbarten Ebene ($E_{L+1}$) mit geringerer Bildauflösung.

**3.** Verfahren nach Anspruch 1 oder 2, wobei die Schritte a bis d für mindestens zwei verschiedene Ebenen ($E_L$, $E_M$) durchgeführt werden und der Schritt e mit mindestens zwei integrierten Maskenbildern ($\Theta_L$, $\Theta_M$) durchgeführt wird.

**4.** Verfahren nach Anspruch 1 oder 2, wobei die Schritte a bis d für eine Vielzahl von Ebenen ($E_i$) der Frequenzzerlegung durchgeführt werden und der Schritt e mit der Vielzahl von integrierten Maskenbildern ($\Theta_i$) durchgeführt wird.

**5.** Verfahren nach einem der vorangehenden Ansprüche, wobei ein Bandpassbild $v_L$ für die erste Ebene mittels der folgenden Formel bestimmt wird:

$$v_L := (\mathbb{I}_L - I_L T_{L+1}^L R_L^{L+1} S_L) \cdot u_L,$$

wobei L die Nummerierung der ersten Ebene, $R_L^{L+1}$ ein auflö- sungsreduzierender Operator, $T_{L+1}^L$ ein auflösungsvergrößernder Operator, S ein Glättungsoperator, I ein Interpolationsopera- $\mathbb{1}_L$ tor, ein Identitätsoperator und $u_L$ das jeweilige Eingangsbild der ersten Ebene sind.

**6.** Verfahren nach einem der vorangehenden Ansprüche, wobei das Gradientenfeld $\nabla v_{L+1}$ des Bandpass- oder Tiefpassbildes der weiteren Ebene, insbesondere der zu der ersten Ebene benachbarten Ebene, hinsichtlich der Bildauflösung des Bandpass- oder Tiefpassbildes angepasst wird, insbesondere unter Verwendung eines auflösungsvergrößernden Operators ( $T_{L+1}^L$ ).

**7.** Verfahren nach einem der vorangehenden Ansprüche, wobei das Skalarprodukt gebildet wird mittels der Formel

$$\langle \nabla v_L, I_L T_{L+1}^L \nabla v_{L+1} \rangle \texttt{ oder } \langle \nabla v_L, I_L T_{L+1}^L \nabla u_{L+1} \rangle,$$

wobei $\nabla v_L$ das Gradientenfeld des Bandpassbildes der ersten Ebene, $\nabla v_{L+1}$ das Gradientenfeld des Bandpassbildes der weiteren Ebene, $T_{L+1}^L$ ein auflösungsvergrößernder Operator, $I_L$ ein Interpolationsoperator und $\nabla u_{L+1}$ das Gradientenfeld des jeweiligen Eingangsbilds der weiteren Ebene sind.

**8.** Verfahren nach einem der vorangehenden Ansprüche, wobei das Maskenbild $\Theta_L$ aus dem Skalarprodukt anhand der folgenden Formel bestimmt wird:

$$\Theta_L := \overline{\langle \nabla v_L, I_L T_{L+1}^L \nabla v_{L+1} \rangle},$$

wobei $\nabla v_L$ das Gradientenfeld des Bandpassbildes der ersten Ebene, $\nabla v_{L+1}$ das Gradientenfeld des Bandpassbildes der weiteren Ebene, $T_{L+1}^L$ ein auflösungsvergrößernder Operator und $I_L$ ein Interpolationsoperator sind.

**9.** Verfahren nach einem der vorangehenden Ansprüche, wobei das Maskenbild ausgebildet ist, eine schärfere Definition von Konturen von in dem medizinischen Bild vorhandenen Kanten zu bewirken.

**10.** Verfahren nach einem der vorangehenden Ansprüche, wobei das Maskenbild ausgebildet ist, eine Glättung von Bereichen des medizinischen Bildes ohne Konturen oder mit wenig ausgeprägten Konturen zu bewirken.

**11.** Verfahren nach einem der vorangehenden Ansprüche, wobei zur Durchführung des Verfahrens zumindest ein maschinenlernender Algorithmus eingesetzt wird.

**12.** Computerprogrammprodukt mit zumindest einem Algorithmus zur digitalen Bildverarbeitung zur Durchführung des Verfahrens nach einem der Ansprüche 1 bis 11.

## Claims

**1.** Computer-implemented method for digital image processing of medical images of an examination object recorded with a medical imaging device, wherein a recorded medical image (10) is decomposed into a plurality of levels ($E_i$) by means of frequency decomposition according to Gaussian-Laplacian decomposition, with the following steps:

a. providing a respective band-pass or low-pass image for calculating an associated gradient field for at least a first level ($E_L$) and for a further level ($E_{L+1}$) of frequency decomposition of the medical image (10),
b. determining a dot product from the gradient field ($\nabla v_L$) of the band-pass or low-pass image of the first level ($E_L$) and the gradient field ($\nabla v_{L+}1$) of the band-pass or low-pass image of the further level ($E_{L+1}$), which is in particular adapted with respect to the image resolution of the band-pass or low-pass image,
c. ascertaining a mask image ($\Theta_L$) for the first level ($E_L$) by normalising the dot product,
d. integrating the ascertained mask image ($\Theta_L$) for the first level ($E_L$) into a parameterisation for processing the band-pass or low-pass image of the first level ($E_L$), and
e. performing an image processing operation with the mask image ($\Theta_L$) integrated into the parameterisation.

**2.** Method according to claim 1, wherein the further level ($E_{L+1}$) is formed by a level ($E_{L+1}$) adjacent to the first level, in particular an adjacent level ($E_{L+1}$) with lower image resolution.

**3.** Method according to claim 1 or 2, wherein steps a to d are performed for at least two different levels ($E_L$, $E_M$) and step e is performed with at least two integrated mask images ($\Theta_L$, $\Theta_M$).

**4.** Method according to claim 1 or 2, wherein steps a to d are performed for a plurality of levels ($E_i$) of frequency decomposition and step e is performed with the plurality of integrated mask images ($\Theta_i$).

**5.** Method according to one of the preceding claims, wherein a band-pass image $v_L$ for the first level is determined by means of the following formula:

$$v_L := (\mathbb{I}_L - I_L T_{L+1}^{L} R_L^{L+1} S_L) \cdot u_L\,,$$

wherein L is the numbering of the first level, $R_L^{L+1}$ is a resolution-downsampling operator, $T_{L+1}^{L}$ is a resolution-upsampling operator, S is a smoothing operator, I is an interpolation operator, $\mathbb{1}_L$ is an identity operator and $u_L$ is the respective input image of the first level.

**6.** Method according to one of the preceding claims, wherein the gradient field $\nabla v_{L+1}$ of the band-pass or low-pass image of the further level, in particular the level adjacent to the first level, is adapted with respect to the image resolution of the band-pass or low-pass image, in particular using a resolution-upsampling operator ( $T_{L+1}^{L}$ ).

**7.** Method according to one of the preceding claims, wherein the dot product is formed by means of the formula

$$\langle \nabla v_L, I_L T_{L+1}^{L} \nabla v_{L+1} \rangle \text{ or } \langle \nabla v_L, I_L T_{L+1}^{L} \nabla u_{L+1} \rangle,$$

wherein $\nabla v_L$ is the gradient field of the band-pass image of the first level, $\nabla v_{L+1}$ is the gradient field of the band-pass image of the further level, $T_{L+1}^{L}$ is a resolution-upsampling operator, $I_L$ is an interpolation operator and $\nabla u_{L+1}$ is the

gradient field of the respective input image of the further level.

**8.** Method according to one of the preceding claims, wherein the mask image $\Theta_L$ is determined from the dot product using the following formula:

$$\Theta_L := \overline{\langle \nabla v_L, I_L T_{L+1}^L \nabla v_{L+1} \rangle},$$

wherein $\nabla v_L$ is the gradient field of the band-pass image of the first level, $\nabla v_{L+1}$ is the gradient field of the band-pass image of the further level, $T_{L+1}^L$ is a resolution-upsampling operator and $I_L$ is an interpolation operator.

**9.** Method according to one of the preceding claims, wherein the mask image is embodied to effect a sharper definition of contours of edges present in the medical image.

**10.** Method according to one of the preceding claims, wherein the mask image is embodied to effect smoothing of regions of the medical image without contours or with poorly pronounced contours.

**11.** Method according to one of the preceding claims, wherein at least one machine-learning algorithm is used to perform the method.

**12.** Computer program product with at least one algorithm for digital image processing for performing the method according to one of claims 1 to 11.

## Revendications

**1.** Procédé mis en œuvre par ordinateur de traitement d'image numérique d'images médicales prises par un appareil d'imagerie médicale d'un objet à examiner, dans lequel on décompose une image (10) médicale prise au moyen d'une décomposition de fréquences suivant une décomposition de Gauss-Laplace dans une pluralité de plans ($E_i$), comprenant les stades suivants :

a. on se procure respectivement une image de bande passante ou de passe-bas pour le calcul d'un champ de gradient associé pour au moins un premier plan ($E_L$) et pour un autre plan ($E_{L+1}$) de la décomposition de fréquences de l'image (10) médicale,
b. on établit un produit scalaire du champ ($\nabla v_L$) de gradient de l'image de bande passante ou de passe-bas du premier plan ($E_L$) par le champ ($\nabla v_{L+}1$) de gradient, adapté en ce qui concerne en particulier la résolution d'image de l'image de bande passante ou de passe-bas, de l'image de bande passante ou de passe-bas de l'autre plan ($E_{L+1}$),
c. on établit une image ($\Theta_L$) de masque pour le premier plan ($E_L$) en normant le produit scalaire,
d. on intègre l'image ($\Theta_L$) de masque établie pour le premier plan ($E_L$) dans un paramétrage de traitement de l'image de bande passante ou de passe-bas du premier plan ($E_L$), et
e. on effectue une opération de traitement d'image avec l'image ($\Theta_L$) de masque intégrée dans le paramétrage.

**2.** Procédé suivant la revendication 1, dans lequel on forme l'autre plan ($E_{L+1}$) par un plan ($E_{L+1}$) voisin du premier plan, en particulier par un plan ($E_{L+1}$) voisin ayant une moindre résolution d'image.

**3.** Procédé suivant la revendication 1 ou 2, dans lequel on effectue les stades a à d pour au moins deux plans ($E_L$, $E_M$) différents et on effectue le stade e avec au moins deux images ($\Theta_L$, $\Theta_M$) de masque intégrées.

**4.** Procédé suivant la revendication 1 ou 2, dans lequel on effectue des stades a à d pour une pluralité de plans ($E_i$) de la décomposition de fréquences et on effectue le stade e avec la pluralité d'images ($\Theta_i$) de masque intégrées.

**5.** Procédé suivant l'une des revendications précédentes, dans lequel on détermine une image $v_L$ réelle de bande passante pour le premier plan au moyen de la formule suivante :

$$v_L := (\mathbb{I}_L - I_L T_{L+1}^L R_L^{L+1} S_L) \cdot u_L,$$

dans laquelle L est la numération du premier plan, $R_L^{L+1}$ un opérateur réduisant la résolution, $T_{L+1}^{L}$ un opérateur augmentant la résolution, S un opérateur de lissage, I un opérateur d'interpolation, $\mathbb{1}_L$ un opérateur d'identité et $u_L$ l'image d'entrée respective du premier plan.

**6.** Procédé suivant l'une des revendications précédentes, dans lequel on adapte, en ce qui concerne la résolution d'image de l'image de bande passante ou de l'image passe-bas, le champ $\nabla v_{L+1}$ de gradient de l'image de bande passante ou de passe-bas de l'autre plan, en particulier du plan voisin du premier plan, en utilisant en particulier un opérateur ( $T_{L+1}^{L}$ ) augmentant la résolution.

**7.** Procédé suivant l'une des revendications précédentes, dans lequel on forme le produit scalaire au moyen de la formule

$$(\nabla v_L, I_L T_{L+1}^{L} \nabla v_{L+1}) \quad \text{ou} \quad (\nabla v_L, I_L T_{L+1}^{L} \nabla u_{L+1}),$$

dans laquelle $\nabla v_L$ est le champ de gradient de l'image passe-bande du premier plan, $\nabla v_{L+1}$ est le champ de gradient de l'image passe-bande de l'autre plan, $T_{L+1}^{L}$ est un opérateur augmentant la résolution, $I_L$ est un opérateur d'interpolation et $\nabla u_{L+1}$ est le champ de gradient de l'image d'entrée respective de l'autre plan.

**8.** Procédé suivant l'une des revendications précédentes, dans lequel on détermine l'image $\Theta_L$ de masque à partir du produit scalaire à l'aide de la formule suivante :

$$\Theta_L := \overline{(\nabla v_L, I_L T_{L+1}^{L} \nabla v_{L+1})},$$

dans laquelle $\nabla v_L$ est le champ de gradient de l'image de bande passante du premier plan, $\nabla v_{L+1}$ est le champ de gradient de l'image de bande-passante de l'autre plan, $T_{L+1}^{L}$ est un opérateur augmentant la résolution et $I_L$ est un opérateur d'interpolation.

**9.** Procédé suivant l'une des revendications précédentes, dans lequel l'image de masque est constituée pour provoquer une définition plus fine des contours de bords présents dans l'image médicale.

**10.** Procédé suivant l'une des revendications précédentes, dans lequel l'image de masque est constituée pour provoquer un lissage de parties de l'image médicale sans contour ou ayant des contours peu marqués.

**11.** Procédé suivant l'une des revendications précédentes, dans lequel on utilise au moins un algorithme d'apprentissage automatique pour effectuer le procédé.

**12.** Produit de programme d'ordinateur comprenant au moins un algorithme pour le traitement de l'image numérique afin d'effectuer le procédé suivant l'une des revendications 1 à 11.

FIG 1
Bereitstellung
Aufgenommenes
Medizinisches Bild
20
Gauß-Laplace-
Frequenzzerlegung in
Vielzahl von Ebenen
21
Bereitstellung Bandpassbilder
und Berechnung
Gradientenfelder erste + weitere Ebene
22
Bestimmung
Skalarprodunkt
23
Ermittlung
Maskenbild
24
Integrierung
Maskenbild
in Parametrierung
25
Bildverarbeitung
26

FIG 2

10

FIG 3

0
50
Θ5
100
150
200
0
50
100
150
200
1.0
0.8
0.6
0.4
0.2
0.0

FIG 4

10
11.1
11.2

FIG 5

38
33
32
31
35.1
35.2

FIG 6

33
32
31
36.1
36.2

FIG 7

$\Theta_L$
33
32
31
37.1
37.2

FIG 8

40
45
41
42
44
43

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2013148871 A1


### In der Beschreibung aufgeführte Nicht-Patentliteratur


- **C. CHO** ; **S. LEE**. Effective Five Directional Partial Derivatives-Based Image Smoothing and a Parallel Structure Design. *IEEE Transactions on Image Processing*, 2016, vol. 25 (4), 1617-1625
- **C. TOMASI** ; **R. MANDUCHI**. Bilateral filtering for gray and color images. *Sixth International Conference on Computer Vision*, 1998, 839-846
- Multiscale vessel enhancement filtering. **FRANGI et al.** Medical Image Computing and Computer-Assisted Intervention - MICCAI'98. Springer Verlag, 1998, vol. 1496, 130-137
- **M. STAHL et al.** Digital Radiography Enhancement by nonlinear multiscale processing. *MEDICAL PHYSICS*, 01 January 2000, vol. 27 (1), ISSN 0094-2405, 56-65
- **P.J. BURT** ; **E.H. ADELSON**. The Laplacian Pyramid as a Compact Image Code. *IEEE Transactions on Communications*, 1983, vol. com-31 (4)